# EUROPEAN PATENT SPECIFICATION

(11) **EP 1 745 805 B1**
(45) Date of publication and mention of the grant of the patent: **13.04.2011**
(21) Application number: 06013927.6
(22) Date of filing: 05.07.2006
(51) Int. Cl.: A61L 2/20, A61L 9/015

(54) **Method for microbiological decontamination and deodorization of areas of motor vehicles**
Verfahren zur mikrobiologischen Dekontaminierung und Deodorierung von Fahrzeugbereichen
Procédé de décontamination microbiologique et déodorisation des régions d' un véhicule

(30) Priority: 19.07.2005 IT VR20050092
(43) Date of publication of application: 24.01.2007
(73) Proprietor: Giugni, Roberto, 37126 Verona (IT)
(72) Inventor: Giugni, Roberto, 37126 Verona (IT)
(74) Representative: Alagem Modiano, Lara S.

(56) References cited:
- JP-A- 2002 191 681
- US-A1- 2005 123 436
- US-A1- 2005 124 286

## Description

The present invention relates to a method for microbiological decontamination and deodorization of areas of motor vehicles and the like, particularly but not exclusively adapted to disinfect and eliminate bad odors in the air circuits of climate control systems installed in motor vehicles and inside the cabins of such motor vehicles.

Several methods are currently known for sanitizing the climate control systems of motor vehicles. Such known methods use products or chemical substances of different kinds, such as foams or vapors, which are usually applied by means of spray cans or vaporizers.

Such sanitizing methods using chemicals have disadvantageous aspects.

First of all, they have a limited effectiveness, since they use active ingredients that have a bland antibacterial action.

Moreover, they have little or no deodorizing action, since they mostly simply cover odors instead of eliminating them permanently, and they suffer the drawback of leaving, after the treatment, chemical residues which in some cases are highly noxious and not easy to remove.

Other currently known solutions combine chemical reagents in order to provide an exothermic reaction, which generates vapors capable of performing a disinfecting action in the circuits of climate control systems.

Although these solutions are certainly effective, they are, however, scarcely practical to perform for a single operator, since it is necessary to combine beforehand the different chemical substances before being able to start the treatment, and these solutions likewise suffer the drawback of producing toxic chemical residues that are difficult to eliminate.

JP-A-2002191681 discloses a method of deodorization and sterilization in which ozone and air are mixed to be ozone air which is directly sprayed to an object through a nozzle to deodorize and sterilize the object.

US2005/0124286 discloses an ozone generating source within a vehicle air-conditioning HVAC unit to decontaminate the vehicle cabin and the HVAC unit.

The aim of the present invention is to solve the problems of the background art described above by providing a method for microbiological decontamination and deodorization of areas of motor vehicles and the like that allows in particular to decontaminate microbiologically and eliminate bad odors in cabins and in the circuits of climate control systems installed in motor vehicles, with a small number of simple operations that can be performed even by a single operator.

Within this aim, an object of the present invention is to provide a method that allows to decontaminate microbiologically and deodorize internal areas of motor vehicles without leaving toxic residues therein after the treatment.

Another object of the invention is to provide a method capable of giving the greatest assurances of effectiveness in terms of results and of safety in use for the operator.

Still another object of the present invention is to provide a decontamination and deodorization method that is competitive also from a merely economical standpoint.

In accordance with the invention, there is provided a method for microbiological decontamination and deodorization of areas of motor vehicles and the like, as defined in the appended claims.

Further characteristics and advantages of the invention will become better apparent from the description of some preferred but not exclusive embodiments of the method according to the invention, illustrated by way of non-limiting example in the accompanying drawings, wherein:

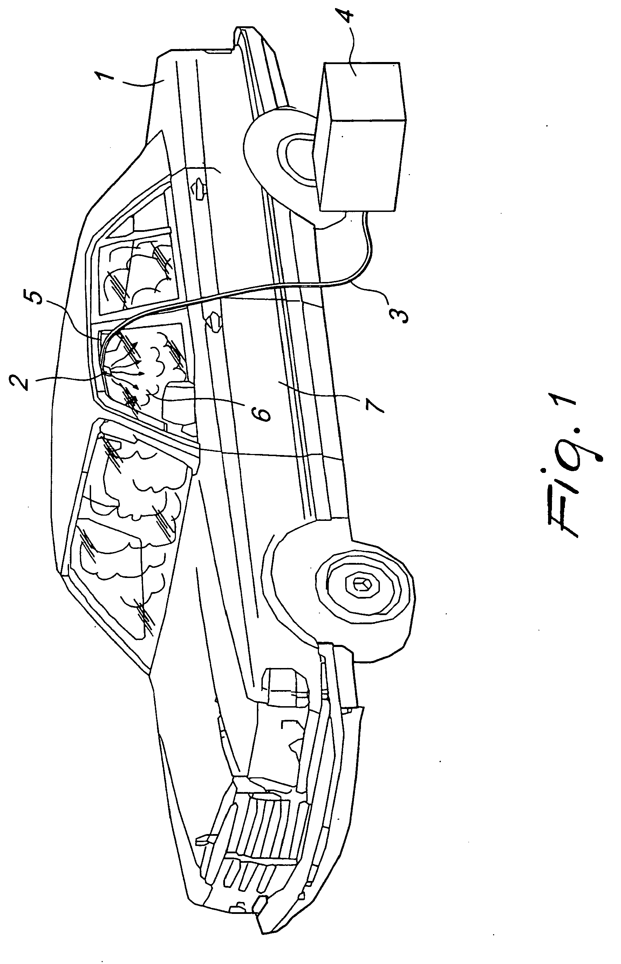

Figure 1 is a schematic view of the execution of the method according to the invention by using an apparatus for generating ozone;

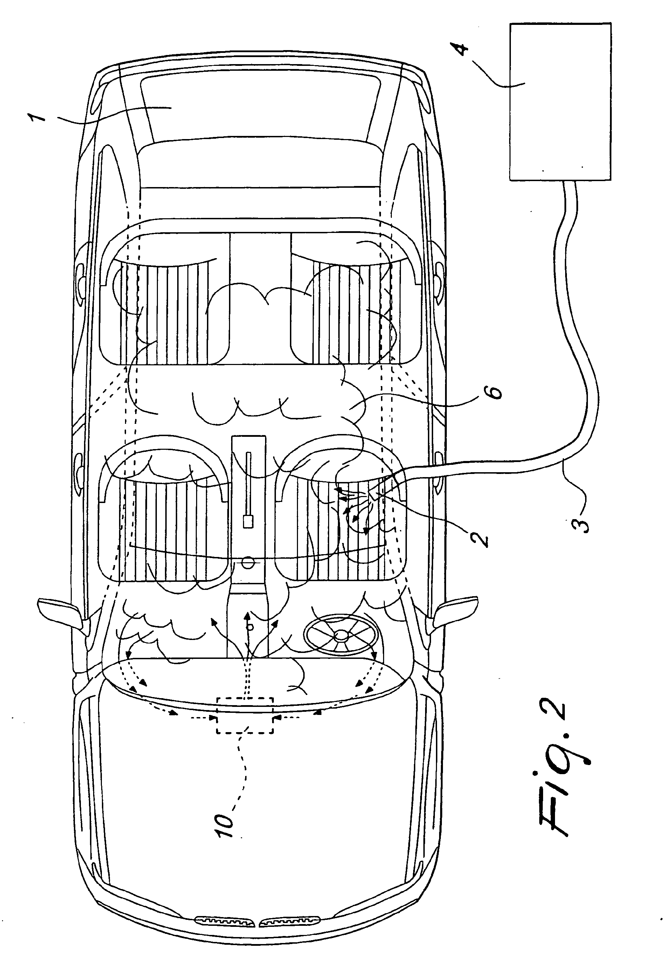

Figure 2 is a schematic plan view, with areas shown in phantom lines, of a motor vehicle during the execution of the method according to the invention, illustrating the operation of its climate control system.

In the exemplary embodiments that follow, individual characteristics, given in relation to specific examples, may actually be interchanged with other different characteristics that exist in other exemplary embodiments.

With reference to the figures, the method for microbiological decontamination and deodorization of the internal areas of motor vehicles and the like, according to the invention, is based substantially on the use of ozone, which has antibacterial properties and leaves no toxic residues of any kind after the treatment.

Therefore, by using ozone in gaseous form it is possible, on one hand, to simplify the treatment operations, since the prior mixing of different chemical substances is not required, and, on the other hand, to ensure a high degree of safety, because of the absence of toxic residues.

Moreover, ozone has the significant advantage of being able to attack the particles that cause bad odors, allowing to truly eliminate them.

In greater detail, the method according to the invention provides for at least one step in which an amount of ozone that can vary according to the level of decontamination and/or deodorization to be achieved is introduced in an area to be treated which is located inside a motor vehicle. In particular, a stream of ozone with a flow-rate that can be selected according to the required results is released in the area to be treated.

Conveniently, in order to carry out the method according to the invention, in practice a nozzle 2 for dispensing a stream of ozone 6 is placed inside the cabin of a motor vehicle 1. In particular, the dispensing nozzle 2 is connected, by means of a flexible duct 3, to a source of ozone 4, which is constituted advantageously by an ozone generator, such as for example the apparatus known by the trade name ZO 103/T, manufactured by the US company Del Industries.

Conveniently, the end of the duct 3 that carries the dispensing nozzle 2 is, for example, inserted in the cabin through a small space 5 left by the pane that closes a window 8 of a side door 7 of the motor vehicle 1.

In this manner it is possible to achieve the microbiological decontamination of the cabin and the elimination of unpleasant odors, such as the smell of smoke or others, which may be present in the cabin.

It should be noted that if the odors are highly localized in a particular region of the cabin, it is convenient to arrange the dispensing nozzle 2 as close as possible to the point of the cabin where the odor is strongest, in order to have a higher concentration of ozone at that point.

Advantageously, during the dispensing of ozone by the dispensing nozzle 2 it is possible to activate, if present, the climate control system, generally designated by the reference numeral 10, in the operating mode with continuous air recirculation, with the diffuser fan preferably operated at its minimum setting, in order to allow the ozone to enter the circuit of the climate control system and accordingly perform its microbiological decontamination.

The treatment performed as described above lasts from 20 to 40 minutes, and the amount of ozone dispensed ranges substantially from 100 to 150 mg, and preferably from 115 to 120 mg. By way of example, it is possible to provide for the substantially constant emission, by the dispensing nozzle 2, of a stream of ozone with a flow-rate ranging substantially from 200 to 250 mg/h for a time substantially ranging from 25 to 40 minutes.

If, after ventilating the cabin adequately, bad odors are found to persist, it is possible to repeat the introduction of ozone until the odor source is completely eliminated, optionally placing the dispensing nozzle 2 at the external air intake of the climate control system and activating the air circuit in the normal mode, with the fan set to minimum.

Substantially, in the practical embodiment of the invention, it is possible to perform even a number of steps for introducing ozone, separated in time by 20-40 minutes, in order to allow, between one step and the next, the perfect execution of the decontaminating activity of the introduced ozone: it should be noted that during each one of such steps it is possible to introduce in the region to be treated an amount of ozone that is conveniently equal to approximately 115-120 mg in a time ranging substantially from 20 to 40 minutes.

It should also be noted that in certain motor vehicles the evaporator of the climate control system is located at a distance from the air circuit, and this causes a less than optimum disinfecting action on the part of the ozone aspirated by the diffuser fan. In such cases, it is convenient to introduce, through the external air intake, the duct 3 for dispensing the ozone inside the duct of the climate control system that reaches the evaporator, blocking if possible the condensation drain in order to avoid the escape of the ozone. In this manner, the stream of ozone dispensed by the duct 3 is capable of striking effectively the evaporator, thus ensuring the perfect elimination of the bacteria and molds that tend to colonize the condensation that collects on the evaporator.

It should also be noted that sometimes, in order to eliminate particularly tenacious odors, it may be necessary, even with the most common climate control systems, to insert the duct 3 in the air supply duct of the circuit of the climate control system, so that the ozone can act effectively in a preset part or even in the entire climate control circuit and more particularly in the region of the evaporator.

It should be noted in this regard that since the ozone is in gaseous form, it can expand easily inside the circuit of the climate control system, so as to come into contact with all the internal surfaces of the climate control system, differently from what foams or other chemical substances used in the background art can do.

The described method, with obvious modifications, can of course be used also to perform the microbiological decontamination and deodorization not only of the cabin and of the circuit of the climate control system but also of other areas inside the vehicle, such as the trunk or the spare wheel receptacle.

In practice it has been found that the method according to the invention fully achieves the intended aim, since thanks to the use of ozone it allows to perform rapidly and safely the suppression of the microbial load in the internal areas of motor vehicles and more particularly in the climate control systems installed therein.

Experimental tests have shown that thanks to the method according to the invention it is possible to reduce the microbial loads present in the internal areas of motor vehicles by an average of 99.9%.

All the characteristics of the invention indicated above as advantageous, convenient or the like may also be omitted or be replaced with equivalents.

The invention thus conceived is susceptible of numerous modifications and variations, all of which are within the scope of the appended claims.

In practice, the materials used, so long as they are compatible with the specific use, as well as the shapes and dimensions, may be any according to requirements.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A method for microbiological decontamination and deodorization of areas of motor vehicles and the like, comprising at least one step consisting in introducing gaseous ozone in a region to be treated of a vehicle, **characterized in that** said at least one step for introducing gaseous ozone is provided in a time interval ranging substantially from 20 to 40 minutes and provides for the dispensing of an amount of gaseous ozone in said region ranging substantially from 100 mg to 150 mg, and **in that** it provides, between one gaseous ozone injection step and the next, a time interval ranging substantially from 20 to 40 minutes.

2. The method according to claim 1, **characterized in that** a nozzle for dispensing a stream of gaseous ozone is arranged substantially at said region to be treated.

3. The method according to claim 1 or 2, **characterized in that** said region to be treated is arranged inside said vehicle and comprises at least one portion of the cabin of said vehicle and/or at least one part of the air circuit of the climate control system of said vehicle.

4. The method according to one or more of the preceding claims, **characterized in that** it provides for the insertion, through an opening provided in said vehicle, of a nozzle for dispensing a stream of gaseous ozone inside the cabin of said vehicle, said dispensing nozzle being connected to a source of gaseous ozone which can be located outside said vehicle.

5. The method according to claim 4, **characterized in that** it provides, during the introduction of said gaseous ozone stream, for the activation of the circuit of the climate control system of said vehicle in the air recirculation mode.

6. The method according to claim 5, **characterized in that** it provides for the insertion of said dispensing nozzle in the air supply duct of the circuit of the climate control system in order to introduce gaseous ozone in at least one part of said circuit.

7. The method according to claim 6, **characterized in that** said at least one part of said circuit comprises the region of the evaporator.

8. The method according to one or more of the preceding claims, **characterized in that** said at least one step for introducing gaseous ozone provides for the dispensing of an amount of gaseous ozone in said region ranging substantially from 115 mg to 120 mg.

## Patentansprüche

1. Verfahren zur mikrobiologischen Dekontaminierung und Geruchsbekämpfung von Gebieten von Kraftfahrzeugen und ähnlichem, das wenigstens einen Schritt umfasst, der aus dem Einleiten von gasförmigem Ozon in eine zu behandelnde Region des Fahrzeugs besteht, **dadurch gekennzeichnet, dass** der wenigstens eine Schritt zum Einleiten von gasförmigem Ozon in einem Zeitintervall vorgesehen ist, der sich im Wesentlichen auf 20 bis 40 Minuten erstreckt und zum Abgeben einer Menge von gasförmigem Ozon in die Region vorgesehen ist, die im Wesentlichen von 100 mg bis 150 mg reicht und **dadurch**, dass es zwischen einem Einleiteschritt von gasförmigem Ozon und dem nächsten einen Zeitintervall vorsieht, der sich im Wesentlichen auf 20 bis 40 Minuten erstreckt.

2. Verfahren nach Anspruch 1, **dadurch gekennzeichnet, dass** eine Düse zum Abgeben eines Stroms aus gasförmigem Ozon im Wesentlichen in der zu behandelnden Region angeordnet wird.

3. Verfahren nach Anspruch 1 oder 2, **dadurch gekennzeichnet, dass** die zu behandelnde Region im Inneren des Fahrzeugs angeordnet ist und wenigstens einen Anteil der Kabine des Fahrzeugs und/oder wenigstens einen Teil des Luftkreislaufs des Klimasteuersystems des Kraftfahrzeugs umfasst.

4. Verfahren nach einem oder mehreren der vorstehenden Ansprüche, **dadurch gekennzeichnet, dass** es das Einführen einer Düse zum Abgeben eines Stroms von gasförmigem Ozon im Inneren der Kabine des Kraftfahrzeuges durch eine Öffnung vorsieht, die in dem Kraftfahrzeug vorgesehen ist, wobei die Abgabedüse mit einer Quelle von gasförmigem Ozon verbunden ist, die außerhalb des Fahrzeugs angesiedelt sein kann.

5. Verfahren nach Anspruch 4, **dadurch gekennzeichnet, dass** es während des Einleitens des Stroms aus gasförmigem Ozon die Aktivierung des Kreislaufs des Klimasteuersystems des Fahrzeuges im Umluftmodus vorsieht.

6. Verfahren nach Anspruch 5, **dadurch gekennzeichnet, dass** es das Einführen der Abgabedüse in den Luftzuführungskanal des Kreislaufs des Klimasteuersystems vorsieht, um gasförmiges Ozon in wenigstens einen Teil des Kreislaufs einzuleiten.

7. Verfahren nach Anspruch 6, **dadurch gekennzeichnet, dass** der wenigstens eine Teil des Kreislaufs die Region des Verdampfers umfasst.

8. Verfahren nach einem oder mehreren der vorstehenden Ansprüche, **dadurch gekennzeichnet, dass** wenigstens ein Schritt zum Einleiten von gasförmigem Ozon die Abgabe einer Menge von gasförmigem Ozon in die Region vorsieht, die im Wesentlichen von 115 mg bis 120 mg reicht.

## Revendications

1. Procédé pour la décontamination microbiologique et la désodorisation de zones de véhicules à moteur et autres, comprenant au moins une étape consistant à introduire de l'ozone gazeux dans un secteur à traiter d'un véhicule, **caractérisé en ce que** ladite au moins une étape pour introduire de l'ozone gazeux est réalisée en un laps de temps compris sensiblement entre 20 et 40 minutes et permet d'introduire une quantité de sensiblement 100 mg à 150 mg d'ozone gazeux dans ledit secteur, et **en ce qu'**il comprend, entre deux étapes successives d'injection d'ozone gazeux, un laps de temps compris sensiblement entre 20 et 40 minutes.

2. Procédé selon la revendication 1, **caractérisé en ce qu'**une buse pour diffuser un flux d'ozone gazeux est disposée sensiblement dans ledit secteur à traiter.

3. Procédé selon la revendication 1 ou 2, **caractérisé en ce que** ledit secteur à traiter est situé à l'intérieur dudit véhicule et comporte au moins une partie de l'habitacle dudit véhicule et/ou au moins une partie du circuit d'air du système de climatisation dudit véhicule.

4. Procédé selon une ou plusieurs des revendications précédentes, **caractérisé en ce qu'**il comprend l'insertion, par une ouverture ménagée dans ledit véhicule, d'une buse pour diffuser un flux d'ozone gazeux à l'intérieur de l'habitacle dudit véhicule, ladite buse de diffusion étant reliée à une source d'ozone gazeux qui peut se trouver à l'extérieur dudit véhicule.

5. Procédé selon la revendication 4, **caractérisé en ce qu'**il comprend, pendant l'introduction dudit flux d'ozone gazeux, l'activation du circuit du système de climatisation dudit véhicule dans le mode circulation d'air.

6. Procédé selon la revendication 5, **caractérisé en ce qu'**il comprend l'insertion de ladite buse de diffusion dans le conduit d'alimentation en air du circuit du système de climatisation afin d'introduire de l'ozone gazeux dans au moins une partie dudit circuit.

7. Procédé selon la revendication 6, **caractérisé en ce que** ladite au moins une partie dudit circuit comporte le secteur de l'évaporateur.

8. Procédé selon une ou plusieurs des revendications précédentes, **caractérisé en ce que** ladite au moins une étape pour introduire de l'ozone gazeux comporte la diffusion d'une quantité de 115 mg à 120 mg d'ozone gazeux dans ledit secteur.
